**Europäisches Patentamt**

**European Patent Office**

**Office européen des brevets**

(11) Veröffentlichungsnummer: **0 127 173**
**A2**

## (12) EUROPÄISCHE PATENTANMELDUNG

(21) Anmeldenummer: **84106101.3**

(22) Anmeldetag: **29.05.84**

(51) Int. Cl.³: **C 12 N 15/00**
**C 12 P 21/00, A 61 K 39/395**

(30) Priorität: **31.05.83 DE 3319751**

(43) Veröffentlichungstag der Anmeldung:
**05.12.84** Patentblatt **84/49**

(84) Benannte Vertragsstaaten:
**AT BE CH DE FR GB IT LI LU NL SE**

(71) Anmelder: **Max-Planck-Gesellschaft zur Förderung der Wissenschaften e.V.**
**Bunsenstrasse 10**
**D-3400 Göttingen(DE)**

(72) Erfinder: **Birchmeier, Walter**
**Vöchtingstrasse 1**
**D-7400 Tübingen(DE)**

(72) Erfinder: **Vollmers, Peter**
**Westbahnhofstrasse 24**
**D-7400 Tübingen(DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel Möhlstrasse 22**
**D-8000 München 80(DE)**

(54) Verfahren zur Gewinnung von gegen Tumorantigene gerichteten monoklonalen Antikörpern und diese enthaltende Arzneimittel.

(57) Es wird ein verfahren zur Gewinnung von gegen Tumorantigene gerichteten monoklonalen Antikörpern beschrieben, bei dem man Inzucht-Versuchstieren bestrahlte Tumorzellen, die sich vom selben Inzuchtstamm ableiten, unter Immunisierungsbedingungen injiziert, danach Milzzellen der so immunisierten Tiere isoliert und unter Anwendung der Hybridomatechnik mit Myelomzellen fusioniert, die erhaltenen Hybridomzellen züchtet und die monoklonalen Antikörper aus dem Kulturüberstand oder aus Aszitesflüssigkeit derjenigen Hybridomaklone gewinnt, welche in vitro die Adhäsion lebender Tumorzellen hemmen oder/und eine Formänderung derselben bewirken. Außerdem wird ein Arzneimittel beschrieben, das als Wirkstoff diese monoklonalen Antikörper neben üblichen pharmazeutischen Zusatz- und Verdünnungsmitteln enthält.

Croydon Printing Company Ltd

# Beschreibung

Die Erfindung betrifft ein Verfahren zur Gewinnung von gegen Tumorantigene gerichteten monoklonalen Antikörpern und ein diese enthaltendes Arzneimittel.

Monoklonale Antikörper sind hochspezifisch reagierende Substanzen, welche nur mit einer bestimmten antigenen Determinante reagieren. Monoklonale Antikörper, welche für Tumorantigene spezifisch sind, die der nicht transformierten Basiszelle des Tumors fehlen, eröffnen neue Möglichkeiten für Therapie und Diagnose von Tumorzellen (transformierten Zellen). Dabei wird die Brauchbarkeit derartiger monoklonaler Antikörper insbesondere für die Krebstherapie letztlich von zwei Faktoren abhängen:

1. ob die Antikörper spezifisch für die Tumorzellen sind und von normalem Gewebe nicht absorbiert werden und

2. ob die Antikörper auch funktionell mit der Tumorentwicklung interferieren. Unter "funktionell aktiv" wird hierbei verstanden, daß die Antikörper, die für die Anhaftung der Tumorzelle an anderen Zellen verantwortlichen Membran-Oberflächenantigene blockiert. Beispielsweise muß ein Antikörper, der lediglich nach seiner Bindungsfähigkeit an Tumorzellen ausgewählt wird, nicht unbedingt auch funktionell aktiv sein.

Im Rahmen der funktionellen Wirksamkeit kommt besonders dem Mechanismus der Metastasenbildung Bedeutung zu. Die Bildung von Metastasen stellt einen kritischen und gleichzeitig auch komplexen Vorgang in der Kanzerogenese
dar. Sie läßt sich in einige verschiedene Schritte aufteilen, beispielsweise Ablösung einiger transformierter
Zellen aus dem Primärtumor, ihre Wanderung im Blutstrom
des Wirtsorganismus, Anheften der angeschwemmten
metastasierenden Tumorzellen an den Endothelien der
Zielstellen, ihre Durchdringung und das Wachsen im
Stroma.

Der Erfindung liegt die Aufgabe zugrunde, monoklonale
Antikörper zu schaffen, welche insbesondere das
Anhaften der metastasierenden Tumorzellen an den
Endothelien spezifisch zu verhindern vermögen.

Gelöst wird diese Aufgabe erfindungsgemäß durch ein
Verfahren zur Gewinnung von gegen Tumorantigene gerichteten monoklonalen Antikörpern, welches dadurch
gekennzeichnet ist, daß man Inzucht-Versuchstieren bestrahlte Tumorzellen, die sich vom selben Inzuchtstamm
ableiten, unter Immunisierungsbedingungen injiziert,
danach Milzzellen der so immunisierten Tiere unter
Anwendung der Hydridomatechnik mit Myelomzellen fusioniert, die erhaltenen Hybridomzellen züchtet und die
monoklonalen Antikörper aus dem Kulturüberstand oder
Aszitenflüssigkeit derjenigen Hybridomaklone gewinnt,
welche in vitro die Adhäsion lebender Tumorzellen
hemmen oder/und eine Formänderung derselben bewirken.

Die Erfindung beruht auf der Erkenntnis, daß eine
Immunisierung von Inzuchtversuchstieren mit bestrahlten
Tumorzellen, die sich vom gleichen Inzuchtstamm ableiten (syngene Tumorzellen) zur Aktivierung von Milzzellen führt, welche Antikörper nur gegen die Oberflächenantigene der Tumorzellen bilden. Diese sind in der nicht
transformierten Basiszelle noch nicht enthalten. Durch
Herstellung von Zellklonen aus derartigen Milzzellen
nach den Methoden der Hybridoma-Technik sind monoklonale Antikörper erhältlich, welche mit menschlichen
Tumorzellen stark kreuzreagieren. Das Verfahren beruht
also darauf, daß Inzuchtversuchstiere mit ihren
"eigenen", syngenen Tumorzellen immunisiert werden und
mit den Milzzellen dieser Tiere Hybridome gebildet und
die aus diesen gezüchteten Klone darauf selektioniert
werden, ob sie monoklonale Antikörper bilden, welche in
vitro die Adhäsion der entsprechenden Tumorzellen auf
künstlichen Substraten hemmen oder/und morphologische
Änderungen (Formänderungen) bewirken und in vivo die
Bildung experimentieller Metastasen bei den Versuchstieren verhindern bzw. das Tumorwachstum behindern.
Wesentlich für die Erfindung ist, daß die für die
Immunisierung verwendeten abgetöteten Tumorzellen
syngen für die verwendeten Inzuchtversuchstiere sind.
Ein Beispiel für derartige syngene Tumorzellen sind die
Melanomzellen der B16- in Bezug auf C57BL/6-Mäuse.
B16-Melanomzellen stellen tumorbildende Mutanten von
C57BL/6-Melanocyten dar.

Die Abtötung der verwendeten syngenen Tumorzellen kann
nach den dem Fachmann hierfür bekannten Methoden erfolgen, beispielsweise durch Einwirkung von Röntgenstrahlen
in geeigneter Dosierung. Wesentlich ist allein, daß die
Oberflächenantigene der Tumorzellen hierbei erhalten
bleiben.

Die Injektion der syngenen Tumorzellen erfolgt unter
den dem Fachmann für die Immunisierung zwecks Antiserumbildung bekannten Methoden. So erfolgt in der Regel
eine mehrfache Immunisierung im Abstand von einigen
Wochen. Bei Verwendung der oben als Beispiel angegebenen
Mäuse und Melanomazellen erwies sich ein Abstand von
zwei bis vier Wochen zwischen den Injektionen als ausreichend, wobei gute Ergebnisse bereits mit zweimaliger
Injektion erhalten wurden. Bei der Immunisierung werden
zweckmäßig übliches Adjuvans zugesetzt wie beispielsweise das komplette oder inkomplette Freund'sche
Adjuvans. Bevorzugt wird die Verwendung von komplettem
Freund'schem Adjuvans für die erste Immunisierung und
von inkomplettem Freund'schem Adjuvans für die zweite
und gegebenenfalls weitere Immunisierung.

Einige Tage nach der letzten Immunisierung werden die
Milzzellen der Versuchstiere gewonnen. Es erwies sich
hierbei ein Zeitraum von zwei bis fünf Tagen als gut
geeignet. Der optimal geeignete Zeitpunkt läßt sich
jedoch durch eine einfache Versuchsreihe leicht bestimmen.

Die Gewinnung der Milzzellen kann operativ ohne Tötung
der Versuchstiere erfolgen. Alternativ werden die
Versuchstiere abgetötet, die Milz entnommen und die
Zellen nach bekannten Methoden separiert.

Durch die syngene Immunisierung erhält man so Milzzellen,
die lediglich gegen den Tumoranteil der für die Immunisierung verwendeten Tumorzellen Antikörper produzieren. Um
die erhaltenen Zellen permanent züchtbar und hinsichtlich
der von ihnen produzierten monoklonalen Antikörper
unterscheidbar zu machen, werden sie nach der bekannten Hybridomatechnik (Köhler und Milstein Nature (London) 256, 495-497 (1975)) mit Myelomzellen fusioniert. Die so erhaltenen Hybridomzellen werden in einem geeigneten Nährmedium unter Bildung von Klonen wachsen gelassen und die überstehende Nährflüssigkeit, in welcher die von jedem Klon gebildeten monoklonalen Antikörper enthalten sind, wird auf ihre Wirksamkeit im in vitro Test zur Verhinderung der Adhäsion lebender Tumorzellen oder zur Formänderung getestet und diejenigen Klone für die Antikörperproduktion ausgewählt, welche die besten Hemmergebnisse bzw. Formänderungen liefern. Für diesen Test werden lebende Tumorzellen der gleichen Tumorart verwendet, die auch für die Immunisierung verwendet wird, also beispielsweise Melanomazellen, wenn mit Melanomazellen immunisiert wurde. Wie sich herausstellte, eignen sich für diese Selektionierung der für die Zellkultur und Produktion von Antikörpern geeigneten Hybridome auch humane Tumorzellen.

Der im Rahmen der Erfindung angewendete Adhäsionstest besteht darin, daß Tumorzellen mit Kulturüberstand der Hybridomzellenklone etwa 1/2 bis 1 Stunde, möglichst unter physiologischen Bedingungen, inkubiert werden. Die Auswertung erfolgt mikroskopisch durch Vergleich mit Kontrollansätzen, bei denen anstelle des Klonkulturüberstands reguläres Kulturmedium oder Überstand der für die Hybridisierung eingesetzten Myelomzelle verwendet wird. Durch visuelle Bestimmung wird dann der Prozentsatz der adhärenten Zellen im Hybridomaüberstand im Vergleich zum Kontrollmedium festgestellt.

Der im Rahmen der Erfindung eingesetzte Adhäsionstest hat gegenüber herkömmlichen Bindungstests mit monoklonalen Antikörpern zwei Vorteile. Zum ersten ist er sehr empfindlich, zum zweiten ist er funktionell. Wie oben
bereits erläutert, werden metastasierende Tumorzellen
im Blut zu fremden Organen geschwemmt, heften sich dort
an den Endothelien an, durchdringen sie und wachsen im
Stroma. Der erste Schritt dieses Vorgangs, nämlich das
Anheften der Tumorzellen an den Zielorganen, wird im
Adhäsionstest in vitro gut simuliert. In vivo Untersuchungen haben ergeben, daß ein erheblicher Teil der
auf diese Weise in vitro aktiven Antikörper auch in
vivo wirksam ist.

Anstelle des Adhäsionstests oder zusätzlich zu diesem
kann auch auf Änderungen der Zellform getestet werden.
Bei dieser Ausführungsform des Tests werden Tumorzellen
mit Kulturüberstand der Hybridomzellenklone etwa 1/2
bis 5 Stunden inkubiert. Die Auswertung erfolgt ebenfalls mikroskopisch durch Vergleich mit Kontrollansätzen
wie beim Adhäsionstest. Diese Ausführungsform des Tests
hat gegenüber herkömmlichen Bindungstests die gleichen
Vorteile wie der Adhäsionstest, läßt sich jedoch noch
schneller durchführen und ermöglicht typischerweise das
Austesten von mehreren hundert verschiedenen Kulturüberständen der Hybridomzellenklone innerhalb weniger
Stunden. Der Test beruht darauf, daß die erfindungsgemäß erhaltenen monoklonalen Antikörper in vielen Fällen
einen drastischen Effekt auf die Morphologie der Tumorzellen ausüben. So werden beispielsweise innerhalb von
1 bis 3 Stunden B16-Melanomzellen rundlich und ihre
dendritenähnlichen Vorsprünge werden stärker ausgeprägt.
TR 126 Humankarzinomzellen dissoziieren auseinander und
CSG120/7 transformierte Mäuse-Speicheldrüsenzellen verklumpen. Neben diesen Wirkungen auf die Zellmorphologie
tritt zumeist auch die oben erläuterte Antiadhäsionswirkung auf.

Weiter wurde gefunden, daß eine Anzahl der gebildeten monoklonalen Antikörper auch neben den oben erwähnten Wirkungen, insbesondere auf die Morphologie der Zellen, auch das Wachstum der Tumorzellen auf Agarplatten hemmt. Es wird angenommen, daß dies darauf zurückzuführen ist, daß diese Antikörper ein normaleres (der gesunden Zelle angeglichenes) Verhalten der Tumorzellen hervorrufen. Dies zeigt sich beispielsweise darin, daß mit diesen monoklonalen Antikörpern behandelte Tumorzellen stärker von Wachstumsfaktoren abhängig werden, während bekanntlich Tumorzellen in Kultur von Wachstumsfaktoren im allgemeinen wenig abhängig sind. Auf nicht transformierte Zellen wird hingegen keine Hemmwirkung entfaltet. Ferner zeigt sich eine Wirkung auf die Bildung der extracellulären Matrix. Beispielsweise erfolgt unter dem Einfluß der erfindungsgemäßen monoklonalen Antikörper eine stärkere Ausbildung von intracellulären und extracellulären faserigen Strukturen. So ist das bei Tumorzellen im allgemeinen wenig ausgeprägte Fibronektinnetz bei den erfindungsgemäß erhaltenen Tumorzellen dem von nicht transformierten normalen Zellen sehr ähnlich.

Die Untersuchung der erfindungsgemäß erhaltenen Antikörper ergab, daß diese mit menschlichen Tumorzellen ebenso stark oder noch stärker reagieren als mit den für die Immunisierung verwendeten syngenen Tumorzellen. Die erfindungsgemäßen Antikörper eignen sich daher zur Prophylaxe und zur Diagnose von Tumoren und Tumorzellen und vermögen insbesondere bei der chirurgischen Tumorentfernung das Risiko des Auftretens von Metastasen durch hierbei ausgeschwemmte Tumorzellen wesentlich herabzusetzen.

Die folgenden Beispiele erläutern die Erfindung.

B e i s p i e l    1

Immunisierung und Produktion der Hybridome

$5x10^6$ bis $10^7$ B16 Maus-melanomazellen (mit 3500 Röntgen
bestrahlt) wurden im Abstand von 3 Wochen in syngene
C57BL/6 Mäuse intraperitoneal injiziert. Für die erste
Immunisierung wurden die Zellen mit 0,5 ml komplettem
Freund's Adjuvans, für die zweite mit inkomplettem
Adjuvans gemischt. 3 bis 4 Tage nach der letzten
Injektion wurden die Tiere getötet und die Milzzellen
nach der Methode von Köhler und Milstein (Nature
(London) 256, 495-497 (1975)) mit NS-1 Myelomazellen
fusioniert.

Nach 10 bis 14 Tagen wurde von den wachsenden Hybridomen
Kulturüberstand entnommen und auf Antikörperaktivität
in funktionellen Tests in vitro untersucht.


In vitro-Adhäsionstest

400 bis 500 B16-Melanomzellen wurden eine Minute mit
Trypsin-EDTA behandelt, die Reaktion durch Zugabe von
überschüssigem Medium, welches 10 % Serum enthält, gestoppt und Zellen bei 200 g abzentrifugiert. Die
abzentrifugierten Zellen wurden mit je 50 µl Hybridomakulturüberstand 30 bis 45 Minuten bei 37°C und 5 % $CO_2$
inkubiert. Als negative Kontrolle dient NS-1 Überstand
oder reguläres Kulturmedium (DMEM* mit 10 % fötalem

---

*DMEM = Dulbecco's modifiziertes Eagle's Medium

Kälberserum). Nach der Inkubation wurden die Gewebskulturschalen mit Phosphatpuffer-haltiger physiologischer Kochsalzlösung gewaschen, die angehefteten Zellen mit 3%igem Formaldehyd fixiert und im Lichtmikroskop ausgezählt, wobei der Prozentsatz der adhärenten Zellen im Hybridomaüberstand im Vergleich zum Kontrollmedium bestimmt wurde.

Test für experimentielle Metastasen

a)  Die die erfindungsgemäßen Antikörper enthaltenden Hybridomaüberstände von 8 Klonen, die wie oben beschrieben funktionell im Adhäsionstest als wirksam festgestellt worden waren, wurden in vitro mit B16 Melanomazellen inkubiert und die Zellen dann syngenen Mäusen nach der Methode von Fidler, Poste und Nicolson intravenös appliziert. Nach 10 bis 12 Tagen wurden die Lungen der Mäuse auf B16-Metastasen untersucht. Dabei ergab sich, daß 40 % der eingesetzten Antikörper im Vergleich zu den entsprechenden Kontrollen die Anzahl der metastasischen Kolonien um über 90 % reduzierten.

b)  Aszitesflüssigkeit der entsprechenden Hybridome wurden 3 bis 4 Stunden vor den B16 Tumorzellen den C57BL/6 Mäusen intravenös oder intraperitoneal gespritzt. Als Kontrolle diente Aszites von NS-1 Hybridomazellen oder von nur in vitro wirksamen Antikörpern. Die Lungen der Tiere wurden wie oben beschrieben auf Metastasen untersucht. Die mit Antikörpern behandelten Tiere zeigten bis zu 80 % weniger experimentielle Metastasen.

Beispiel    2

Die wie im Beispiel 1 beschrieben hergestellten monoklonalen Antikörper wurden auf ihre Adhäsivität in vitro
gegenüber 10 verschiedenen menschlichen Melanomzellinien
getestet. Die verwendeten menschlichen Melanomzellinien
und die erhaltenen Ergebnisse im Vergleich zur Kontrollprobe sind in der nachstehenden Tabelle 1 angegeben.

Adhäsionshemmung im Vergleich zur Kontrolle

# T a b e l l e   1

Adhäsionshemmung im Vergleich zur Kontrolle

| Antikörper | A357 | SK-Mel-25 | Strömer | MelJuSo | IGR3 | ParL | MelWei | MelHo | MelIm | MelJu |
|---|---|---|---|---|---|---|---|---|---|---|
| 1 | ++++ | ++++ | ++++ | ++++ | ++++ | +++ | ++ | + | + | - |
| 2 | ++++ | ++++ | ++++ | ++++ | +++ | +++ | ++ | + | - | - |
| 3 | ++++ | ++++ | +++ | +++ | ++ | - | + | - | + | - |
| 4 | ++++ | ++++ | +++ | +++ | ++ | + | - | - | - | - |
| 5 | ++++ | ++++ | +++ | +++ | ++ | - | - | - | - | - |
| 6 | ++++ | ++++ | +++ | +++ | ++ | + | - | - | - | - |
| 7 | ++++ | ++++ | +++ | ++ | ++ | + | - | - | - | - |
| 8 | ++++ | +++ | +++ | ++ | + | + | - | - | - | - |

++++ = 80 bis 100 % Hemmung der Adhäsion;

+++ = 60 bis 80 %;

++ = 40 bis 60 %;

+ = 20 bis 40 %;

- = 0 bis 20 %.

0127173

Die erfindungsgemäßen Antikörper, die unter Verwendung
von Melanomzellen gebildet worden waren, kreuzreagieren
nicht nur mit menschlichen Melanomzellen sondern auch
mit anderen menschlichen Tumorzellen. Keine Kreuzreaktion
findet jedoch statt mit nicht transformierten menschlichen
Zellen (embryonale Lungenfibroblasten MRC-5).

Die verwendeten Tumorzellen und die erhaltenen
Ergebnisse im Adhäsionstest sind in der nachstehenden
Tabelle 2 gezeigt.

### T a b e l l e    2

Adhäsionshemmung im Vergleich zur Kontrolle

| Antikörper | HeLa | TuWi[1] | A549[2] | A431[3] | Tagli-glio-blastom | MRC-5[4] |
|---|---|---|---|---|---|---|
| 1 | +++ | ++++ | ++++ | +++ | ++++ | - |
| 2 | +++ | +++ | ++++ | ++ | +++ | - |
| 3 | ++ | ++ | +++ | ++ | ++ | - |
| 4 | ++++ | ++ | +++ | ++ | ++ | - |
| 5 | ++ | ++ | +++ | ++ | ++ | - |
| 6 | +++ | + | +++ | ++ | +++ | - |
| 7 | + | + | + | + | ++ | - |
| 8 | - | + | ++ | ++ | - | - |

Die Bewertung erfolgt wie in Tabelle 1.

[1] Wilms Tumor,
[2] Lungencarcinom,
[3] Vulvacarcinom,
[4] normaler embryonaler Lungenfibroblast

B e i s p i e l      3

C57BL/6 Mäuse wurden wie im Beispiel 1 beschrieben, mit
B16-FL-Melanomazellen immunisiert, jedoch mit ·
dreimaliger Injektion. Die Milzzellen wurden dann nach
der Methode von Köhler und Milstein mit NS-1 Myelomazellen fusioniert. Zur Isolierung der monoklonalen
Antikörper wurden die Hybridome in Rollflaschen in DMEM
mit einem Gehalt von 10 % fötalem Kälberserum

Zum Austesten der Hybridomüberstände wurden frisch
trypsinisierte Tumorzellen (gewöhnlich C7, ein epithelähnlicher B16 Melanomklon, oder TR126, ein Humanzungenkarzinom, oder CSG120/7, eine chemisch transformierte
Mäusespeicheldrüsen-Epitelzelle) auf Terasakiplatten
gebracht und zwar in einer Menge von 3000 bis 5000
Zellen pro Vertiefung. Nachdem sich die Zellen ausgebreitet und annähernd zusammengewachsen waren (einige
Stunden) wurde das Medium durch 30 µl Hybridoma-Überstand pro Vertiefung ersetzt und die Platten bei 37°C
inkubiert. NS-1 Überstand diente als Kontrolle. Nach 1
bis 4 Stunden wurden die Platten mit 3 % Formaldehyd
fixiert und in einem Umkehrmikroskop auf Dissoziation
und Änderung der Zellform kontrolliert. Klone, die eine
deutlich erkennbare Änderung der Zellmorphologie
bewirkten, wurden isoliert und mit 2 dieser Klone, die
als Norm-1 und Norm-2 bezeichnet wurden, erfolgten die
weiteren Untersuchungen.

Die in vitro Wirksamkeit dieser monoklonalen Antikörper
Norm-1 und Norm-2 auf verschiedene tierische und
menschliche Zellinien in bezug auf die Beeinflussung
der Morphologie und der Adhäsion zeigt nachstehende
Tabelle 3.

# T a b e l l e   3

| Zellinie | Wirkung auf Morphologie | | Adhäsion | |
|---|---|---|---|---|
|  | Norm-1 | Norm-2 | Norm-1 | Norm-2 |
| **Tierzellen:** | | | | |
| B16 Maus Melanom | ++ | ++++ | ++ | ++ |
| CSG 120/7 trans-formierte Maus-Epithelzellen | +++ | ++ | ++ | ++ |
| F9 Maus Terato-carcinom | + | ++ | ++ | +++ |
| 3T3 Maus Fibroblasten | − | − | − | − |
| Rinderaorta-endothelial-zellen | − | | | |
| **Humanzellen:** | | | | |
| Strömer Melanom | ++++ | ++ | ++++ | ++ |
| MelHO Melanom | − | ++++ | − | − |
| HeLa Carcinom | ++ | ++++ | ++ | ++++ |
| A431 Carcinom | ++ | ++++ | ++ | ++++ |
| TR126 Carcinom | ++ | + | ++ | +++ |
| Wilm Tumor | ++ | + | ++ | ++++ |
| MRC5 Fibroblasten | − | − | − | − |

Die Wirkung auf die Morphologie wurde visuell gewertet.

++++ bedeutet sehr starke Wirkung

− bedeutet keine Wirkung.

In den Adhäsionsversuchen bedeutet

++++ 80 bis 100 % Hemmung,

+++ 60 bis 80 %,

++ 40 bis 60 %,

+ 20 bis 40 % und

− 0 bis 20 % Hemmung.

Bei den 3T3 Fibroblasten, den MRC5 Fibroplasten und den
Rinderendothelialzellen handelt es sich um nicht
transformierte Zellen, alle übrigen sind bekannte
Tumorzellinien.

Die Wirksamkeit der erfindungsgemäßen Antikörper Norm-1
und Norm-2 auf das Wachstum von metastatischen Lungentumoren des B16 Melanoms bei nachträglicher Behandlung
zeigt die nachstehende Tabelle 4.


T a b e l l e    4


| Antikörper-behandlung | sichtbare Lungenläsionen Zahl | Mittelwert | % |
|---|---|---|---|
| Kontrolle | 102,113,139,141,224 | 144 | 100 |
| Norm-1 | 1,1,5,12,15,17,19,22,45,45 | 18 | 13 |
| Kontrolle | 13,25,27,43,46,62,73,94,151 | 59 | 100 |
| Norm-2 | 2,4,6,11,11,14,16,20 | 11 | 19 |
| Kontrolle | 88,109,120,122,154,160 | 126 | 100 |
| 36/74 | 9,65,86,97,183,230 | 112, | 89 |

$10^5$ hochmetastatische B16 Melanomzellen wurden erst
intravenös in C57BL/6 Mäuse injiziert. 3 Tage später
wurden die Antikörper intraperitoneal verabreicht,
entweder in Form von 100 µl Ascitesflüssigkeit oder als
1,5 Verdünnung in phosphatgepufferter physiologischer
Kochsalzlösung. Nach 10 bis 12 Tagen wurden die Lungen
visuell auf die experimentiellen Metastasen untersucht.

Als Kontrolle dient Ascitesflüssigkeit von NS-1 Myelom.
Antikörper 36/74 zeigte im morphologischen Test eine
ähnliche Wirkung auf die Tumorzellenform wie Norm-1 und
Norm-2, jedoch keinen normalisierenden Einfluß in
vitro.

<u>P a t e n t a n s p r ü c h e</u>

1.  Verfahren zur Gewinnung von gegen Tumorantigene
    gerichteten monoklonalen Antikörpern,
    d a d u r c h        g e k e n n z e i c h n e t ,
    daß man Inzucht-Versuchstieren bestrahlte Tumorzellen, die sich vom selben Inzuchtstamm ableiten,
    unter Immunisierungsbedingungen injiziert, danach
    Milzzellen der so immunisierten Tiere isoliert und
    unter Anwendung der Hybridomatechnik mit Myelomzellen fusioniert, die erhaltenen Hybridomzellen
    züchtet und die monoklonalen Antikörper aus dem
    Kulturüberstand oder aus Aszitesflüssigkeit
    derjenigen Hybridomaklone gewinnt, welche in vitro
    die Adhäsion lebender Tumorzellen hemmen oder/und
    eine Formänderung derselben bewirken.

2.  Arzneimittel,           d a d u r c h
    g e k e n n z e i c h n e t ,       daß es als
    Wirkstoff monoklonale Antikörper gemäß Anspruch 1
    bis 4 neben üblichen pharmazeutischen Zusatz- und
    Verdünnungsmitteln enthält.